# EUROPEAN PATENT APPLICATION

(11) **EP 2 778 682 A1**
(43) Date of publication of application: **17.09.2014**
(21) Application number: 12848691.7
(22) Date of filing: 08.11.2012
(51) Int. Cl.: G01N 33/579, G01N 21/64, G01N 21/82

(54) **METHOD FOR MEASURING PHYSIOLOGICALLY ACTIVE SUBSTANCE OF BIOLOGICAL ORIGIN**

(30) Priority: 10.11.2011 JP 2011246957
(71) Applicant: Kowa Company, Ltd., Nagoya-shi, Aichi 460-8625 (JP)
(72) Inventor: YABUSAKI, Katsumi, Tokyo 103-8433 (JP)
(74) Representative: Chettle, Adrian John
(86) International application number: PCT/JP2012/079037
(87) International publication number: WO 2013/069749

(57) **Abstract**

Provided is a technique which enables the detection of a physiologically active substance of biological origin or the measurement of the concentration of the physiologically active substance with higher accuracy and also enables the visual observation of the process of the aggregation or gelatinization of a protein, which is caused by the reaction between an AL and the physiologically active substance in a method for measuring the physiologically active substance. A method for measuring a specific physiologically active substance of biological origin, comprising preparing a mixed solution of an AL reagent and a sample containing the physiologically active substance and detecting the aggregation or gelatinization of a protein in the mixed solution while agitating the mixed solution, thereby detecting the physiologically active substance or measuring the concentration of the physiologically active substance in the sample. A function of emitting a predetermined fluorescence is imparted to a substance involved in the aggregation or gelatinization in the sample to be measured and/or the AL reagent, and the fluorescence emitted from the substance involved in the aggregation or gelatinization is measured or observed in the mixed solution. In this manner, the aggregation or gelatinization in the mixed solution can be detected.

## Description

### Technical Field

The present invention relates to a measurement method for detecting a physiologically active substance of biological origin having a property of being aggregated or being gelated by reaction with AL such as endotoxin and β glucan in a sample containing the physiologically active substance, or measuring the concentration thereof.

### Background Art

Endotoxin is a lipopolysaccharide present in a cell wall of a Gram-negative bacterium and is the most typical pyrogen. If a transfusion, a medicine for injection, the blood or the like contaminated with endotoxin is introduced into the human body, endotoxin may induce severe side effects such as fever and shock. Therefore, it has been required that the above-mentioned medicine or the like is kept not to be contaminated with endotoxin. On the other hand, measurement of endotoxin in the blood of a patient having sepsis may contribute to prevention or treatment of severe endotoxin shock.

In addition, β glucan is a polysaccharide that constructs the cell membrane that is characteristic to a fungus. Measurement of β glucan is effective for screening of infectious diseases due to a variety of fungi including not only fungi which are frequently observed in general clinical practices such as Candida, Aspergillus, and Cryptococcus, but also rare fungi, and the like.

By the way, serine protease activated by endotoxin, β glucan or the like exists in a hemocyte extract of limulus (hereinafter, also referred to as "AL: Amoebocyte lysate".). Then, when AL reacts with endotoxin or β glucan, a coagulogen existing in AL is hydrolyzed to a coagulin by an enzyme cascade by the serine protease activated according to the amount of endotoxin or β glucan, and the coagulin is associated to form an insoluble gel. With the use of this property of AL, it is possible to detect endotoxin or β glucan with high sensitivity. In recent years, with use of this, methods of using AL in detection or concentration measurement of endotoxin and the like were invented.

As a method for performing detection or concentration-measurement of this physiologically active substance of biological origin (hereinafter, also referred to as "the predetermined physiologically active substance".) detectable by AL such as endotoxin and β glucan (hereinafter, also simply referred to as the "measurement of the predetermined physiologically active substance".), the followings are known. For example, the method includes a turbidimetric method including analyzing a sample by measuring the turbidity of the sample by the reaction between AL and the predetermined physiologically active substance; a colorimetric method using a synthetic substrate that is hydrolyzed by an enzyme in AL activated by the predetermined physiologically active substance to develop a color; and the like. However, the turbidity of AL itself or the coloring by a synthetic substrate when a low concentration of the predetermined physiologically active substance is applied, is extremely weak, and thus it could not be said that the lower limit for measuring the physiologically active substance in these measurement methods was always sufficient.

On the other hand, the measurement sensitivity of some of dialysis fluids is insufficient with a conventional turbidimetric method or a colorimetric method. Thus, establishment of a measurement method for achieving further higher sensitivity is urgently needed. As the measurement method for achieving the higher sensitivity, reported are a method developed from the turbidimetric method in which the sample is stirred (stirring turbidimetric method), a method in which gel microparticles formed in a stirred sample are detected with light scattering method (light scattering method), a method in which AL is bonded onto the microparticles whereby to reinforce the aggregation reaction (AL-bound bead method), and the like.

However, in the method for measuring the predetermined physiologically active substance, the measurement is performed in a lighted room using a laser light source or LED light source as a light source, and thus the accuracy of the measurement may decrease depending on the influence of surrounding light. In addition, it was difficult to visually observe the process of aggregation or gelation based on the reaction between the predetermined physiologically active substance in a sample and AL in an AL reagent.

### Citation List

### Patent Literature

Patent Literature 1: Japanese Patent Application Laid-Open (JP-A) No. 2009-150723
Patent Literature 2: WO 2008/038329 A

### Summary of Invention

### Technical Problem

The present invention has been developed in view of the above-mentioned problems. An object of the present invention is to provide a technology for achieving more accurate detection and concentration measurement of a predetermined physiologically active substance, and visual observation of the process of protein aggregation or gelation induced by the reaction between AL and the predetermined physiologically active substance, wherein the technology is a method for measuring a physiologically active substance of biological origin by: producing a mixed solution of an AL reagent containing AL which is a hemocyte extract of limulus and a sample containing the predetermined physiologically active substance, and detecting the protein aggregation or gelation induced by the reaction between AL and the predetermined physiologically active substance in the mixed solution while stirring the mixed solution, whereby to detect the predetermined physiologically active substance or measure the concentration of the predetermined physiologically active substance in the sample.

### Solution to Problem

To solve the above-mentioned problems, the present invention provides a method for measuring a physiologically active substance of biological origin by: producing a mixed solution of a sample containing the predetermined physiologically active substance and an AL reagent, and detecting protein aggregation or gelation in the mixed solution while stirring this mixed solution, whereby to perform detection or concentration-measurement of the physiologically active substance in the sample. Then, the greatest feature of the present invention is imparting a function of generating a predetermined fluorescence to a substance involved in the aggregation or gelation in a subject sample to be measured and/or an AL reagent, and measuring or observing the fluorescence of the substance involved in the aggregation or gelation in the mixed solution whereby to perform detection of the aggregation or gelation in the mixed solution.

More specifically, the present invention provides a method for measuring a physiologically active substance of biological origin by producing a mixed solution of an AL reagent containing an AL which is a hemocyte extract of limulus and a sample containing a predetermined physiologically active substance of biological origin, and detecting protein aggregation or gelation induced by the reaction between AL and the physiologically active substance in the mixed solution while stirring the mixed solution, whereby to detect the physiologically active substance in the sample or measure the concentration of the physiologically active substance, which is characterized by
imparting a function of generating a predetermined fluorescence to a substance involved in the aggregation or gelation in the sample and/or the AL reagent, and binding a dye to the substance, and
measuring or observing the fluorescence from the substance involved in the aggregation or gelation in the mixed solution whereby to detect the aggregation or gelation in the mixed solution, and thus detect the physiologically active substance or measure the concentration of the physiologically active substance in the sample.

In other words, in the present invention, a function of generating a predetermined fluorescence is imparted to a substance involved in protein aggregation or gelation in a sample containing the predetermined physiologically active substance and/or an AL reagent. In that case, it is possible to visually observe the mechanism of the aggregation or gelation by observing the state of the fluorescence from the substance involved in the protein aggregation or gelation when the protein aggregation or gelation occurs. In addition, it is possible to detect the protein aggregation or gelation by measuring the fluorescence intensity since the fluorescence intensity increases at a site where the aggregation or gelation is generated.

Furthermore, in the case where fluorescent dyes are close to each other, exchange of the energy with each other between the fluorescent dyes occurs, and close of the fluorescence tends to increases. Accordingly, in the present invention, when the fluorescent dyes are close to each other by the aggregation or gelation, the fluorescence strength from the portion increases, which makes it possible to detect the protein aggregation or gelation induced by the reaction between AL and the predetermined physiologically active substance with higher sensitivity. In addition, when the fluorescence or the fluorescence intensity is measured, the surrounding is usually made dark. Accordingly, in the present invention, it is possible to reduce the background noise, and it is possible to detect the protein aggregation or gelation induced by the reaction between AL and the predetermined physiologically active substance with higher accuracy.

Furthermore, in the present invention, the substance involved in the aggregation or gelation in the sample and/or the AL reagent includes proteins contained in AL such as coagulogen, and in addition, the beads in the AL-bound bead method and microparticles having adsorbed endotoxin.

In addition, the present invention provides a method for measuring a physiologically active substance of biological origin by binding the predetermined protein contained in AL to a bead dispersible in the AL reagent,
mixing AL-bound bead reagent which is an AL reagent in which the beads bound with the protein are dispersed with the sample and
detecting the protein aggregation or gelation in the mixed solution of the AL-bound bead reagent and the sample whereby to perform detection or concentration measurement of the physiologically active substance, wherein
a predetermined fluorescent dye is previously bonded to the bead, or the bead is formed with the predetermined inorganic fluorescent dye, whereby to impart a function of generating a predetermined fluorescence to the bead.

Herein, it is understood that if the protein contained in AL is bonded to the bead and in this state, the protein is applied with the predetermined physiologically active substance, a larger aggregation mass is early produced in comparison to the case where the predetermined physiologically active substance is applied to a simple substance of AL. This is because the protein on the bead (for example, coagulogen) hydrolyzed by the enzyme cascade in AL to a coagulin, and the coagulin is aggregated or gelated by itself and also make the beads associated with each other.

In the present invention, the function of generating a predetermined fluorescence may be imparted to the bead by previously binding a fluorescent dye to this bead, or forming the bead with a predetermined inorganic fluorescent dye. According to this, it is possible to early produce a larger aggregation mass and detect or observe the production of this aggregation mass with the fluorescence in comparison to the case where a simple substance of AL and/or the predetermined physiologically active substance is imparted with a function of generating the predetermined fluorescence, and they are mixed. Furthermore, in the case where the bead is porous, or in the case where nano-primary particles are associated to a large secondary particle, the particle is tagged with fluorescence not only on the surface but also in the inside when the particle is bonded with the fluorescent dye. Accordingly, binding a fluorescent dye to the bead in the present invention includes binding the fluorescent dye onto the surface of the bead, and binding the fluorescent dye onto the inside of the bead.

In addition, the predetermined fluorescent dye in the present invention is not particularly limited, but conventionally used fluorescent dyes in dyeing a biomolecule may be suitably used, which may include fluoresceins, rhodamines, coumarins, pyrenes and cyanines.

In addition, as the predetermined fluorescent dye, 4',6-diamidino-2-phenylindole (DAPI), umbelliferone, 9,10-bis(phenylethynyl)anthracene, 2-chloro-9,10-bis(phenylethynyl)anthracene, SYBR Green (SG, SYBR Green), ethidium bromide, stilbene, Nile blue (Nile blue A), 5,12-bis(phenylethynyl)naphthacene, pyranine, perylene, boron-dipyrromethene, merocyanine, green fluorescence protein (GFP), luciferin, rubrene, Lumazine protein (LumP) and the like may be used. In addition, fluorescein isothiocyanate (FITC), rhodamine isothiocyanate (RITC), QUARTZ DOT (trademark) that is a fluorescent dye in which an organic dye molecule is immobilized in silica (silicon dioxide) particles, or the like may be used.

In addition, examples of the inorganic fluorescent dye may include, for example, ZnS: Ag, (ZnCd)S: Cu, (ZnCd)S: Ag, Zn2SiO4: Mn, Cd2B2O5: Mn, (SrMg)3(PO4)2: Mn, YVO3: En, CaWO4, Y2O2: Eu, Y2O2: Mg, Y2O2: Ti, ZnS: Ag, ZnS: Al, ZnS: Cu, ZnS: Al, Y2O2S: Eu, (SrCaBaMg)5(PO4)3Cl: Eu, LaPO4: Ce, LaPO4: Tb, Y2O3: Eu, CalO(PO4)6FCl: Sb, CalO(PO4)6FCl: Mn, BaMgA110O17: Eu, Zn2SiO4: Mn, YBO3: Eu, GdBO3: Eu, CaWO4, Gd2O2S: Tb, YTaO4: Nb, SrTaO4: Nb, Y202: Eu, Y202: Mg, Y202: Ti and the like (the enumerated inorganic fluorescent dyes herein are also referred to as the inorganic fluorescent dye group below). Furthermore, the inorganic fluorescent dye group may be used to be bonded to the substance involved in the aggregation or gelation, and in addition, may be also used as the bead itself emitting fluorescence. Accordingly, by forming the bead with the inorganic fluorescent dye group, it is possible to impart a function of generating a predetermined fluorescence to the bead.

In addition, the predetermined protein described above is those having aggregation or gelation property by the reaction with the predetermined physiologically active substance such as endotoxin, and at least includes coagulogen. In addition, the predetermined protein may include other proteins such as serine protease. In addition, the gelation (production of gel) in the present invention means at least any one phenomena of production of an aggregation mass by association of the beads with each other, production of gel particles by association of coagulin not bonded or adsorbed with the bead, and gelation of a mixed solution of the AL reagent and the sample.

In addition, the bead is selected from those capable of adsorbing, supporting or binding the proteins in AL (for example, coagulogen), being uniformly dispersed in the reaction system, and not preventing or promoting the gel production process by the predetermined physiologically active substance. The material for the bead is not particularly limited. Example of the material for the bead may include alumina, titania, polystyrene latex resin, silica, a silicon resin, a cellulose resin, a polyvinyl alcohol resin, hydroxyapatite and the like, and may include alumina, titania, a polystyrene latex resin, silica and a cellulose resin in the case where the bead is bound with a fluorescent dye other than the inorganic fluorescent dye group.

The size of the bead used is in a range of 0.05 µm to 50 µm in view of conditions enabling early detection or observation by the aggregation, easiness of the handling at the time of the preparation, easiness of dispersion in the system, and the like. Then, the size of the bead is further desirably in a range of 0.1 µm to 30 µm. As a method for binding the predetermined physiologically active substance to the bead, considered are a method of adsorbing the predetermined physiologically active substance electrostatically, hydrophilically or hydrophobically, and a method of chemically binding the predetermined physiologically active substance.

In addition, in the present invention, the predetermined fluorescent dye may be bonded to the protein that is aggregated or gelated induced by the reaction between AL and the physiologically active substance in the mixed solution, whereby to impart a function of generating the predetermined fluorescence to the protein. In other words, the predetermined fluorescent dye may be bonded to the protein that is aggregated or gelated induced by the reaction between AL and the physiologically active substance in the mixed solution instead of the predetermined fluorescent dye being bonded to the bead, or in addition to the predetermined fluorescent dye being bonded to the bead in the measurement using the AL-bound bead reagent, whereby to reflect more specifically the mechanism of the aggregation or gelation in the fluorescence distribution or the fluorescence amount.

As a result, it is possible to measure the predetermined physiologically active substance more accurately, and more specifically, visually observe the mechanism of the aggregation or gelation. In addition, by binding the predetermined fluorescent dye to the protein that is aggregated or gelated induced by the reaction between AL and a physiologically active substance, it is possible to measure a predetermined physiologically active substance more accurately, and more specifically, visually observe the mechanism of the aggregation or gelation even in the case where the AL-bound bead reagent is not used and the measurement of the predetermined physiologically active substance is performed with mixing the AL reagent with the sample containing the predetermined physiologically active substance.

Furthermore, the predetermined protein may be a coagulogen purified from the hemocyte extract of limulus. Then, it is possible to more specifically observe the processes where a coagulation enzyme is produced by the reaction of the predetermined physiologically active substance and AL, this coagulation enzyme hydrolyzes the coagulogen in AL to produce a coagulin, the produced coagulins are associated together to further produce an insoluble gel, and the whole AL is involved with this and gelated, and it is possible to perform more accurate measurement of the predetermined physiologically active substance in the processes.

In addition, the present invention provides a method for measuring a physiologically active substance of biological origin by dispersing microparticles capable of adsorbing the physiologically active substance in the sample whereby to adsorb the physiologically active substance onto the microparticles and then isolate the microparticles from the sample,
producing a mixed solution of the AL reagent and the microparticles adsorbed with the physiologically active substance,
detecting aggregation or gelation of the microparticles in the mixed solution of the AL reagent and the microparticles, wherein the method includes
binding the predetermined fluorescent dye to the microparticle in advance, or forming the microparticle with the predetermined inorganic fluorescent dye, whereby to impart a function of generating the predetermined fluorescence to the microparticle.

In this case, microparticles containing a material having sufficient strength against mechanical external force are mixed with a sample containing the predetermined physiologically active substance, to adsorb the predetermined physiologically active substance onto the microparticle. The sample at the time may have a property that the concentration of the predetermined physiologically active substance is extremely low and/or a property of containing an interference substance affecting the reaction of AL. Then, the microparticles adsorbed with the predetermined physiologically active substance, are isolated and collected by spontaneous sedimentation or an operation such as centrifugal isolation or filtration by a filter, and as necessary, the microparticles are washed with a liquid not containing the predetermined physiologically active substance. Then, the microparticle and AL are reacted whereby to induce the aggregation reaction, and measurement of the predetermined physiologically active substance is performed.

Then, in the present invention, the microparticles may be imparted with a function of generating a predetermined fluorescence by being bound with a fluorescent dye in advance, or being formed with a predetermined inorganic fluorescent dye. According to this, it is possible to concentrate the predetermined physiologically active substance in the sample on the microparticles, and as a result, it is possible to perform more accurate detection or concentration-measurement of the physiologically active substance of biological origin in a shorter time by fluorescence. In addition, it is possible to observe the aggregation or gelation of the microparticles in the mixed solution of the AL reagent and the microparticles in a shorter time by fluorescence.

Furthermore, herein, the microparticles are also tagged with fluorescence not only on the surface, but also onto the inside of the particle when the microparticles are bound with a fluorescent dye in the case where the microparticles are porous, or in the case where nanoprimary particles are associated to a large secondary particle. Accordingly, binding a fluorescent dye to the microparticle in the present invention includes binding the fluorescent dye onto the surface of the microparticle, and binding the fluorescent dye onto the inside of the microparticle.

The microparticle in the present invention may be composed of one or multiple materials selected from PyroSep, polymixin B, polylysine, polyornithine, polyarginine, polyhistidine, aminosilane, chitosan, an anti-endotoxin antibody, an anti-endotoxin aptamer, a material in a random peptide library, a metal oxide such as alumina, titania, silica, zirconia and hydroxyapatite, and a natural or synthetic mineral such as kaolin, montmorillonite, manganese oxide and mica.

In other words, the adsorption material for adsorbing the predetermined physiologically active substance may be either an organic compound having excellent adsorption ability for the predetermined physiologically active substance such as endotoxin and having high selectivity, or an inorganic substance not having high selectivity but having excellent adsorption ability for the predetermined physiologically active substance such as endotoxin. Examples of the organic compound include PyroSep, polymixin B, polylysine, polyornithine, polyarginine, polyhistidine, aminosilane, chitosan, an anti-endotoxin antibody, an anti-endotoxin aptamer, an endotoxin adsorption peptide selected from a random peptide library (for example, XYSSS (X = K, R, or H) exemplified in JP-A No. 2009-118854), and the like. In addition, examples of the inorganic substance include a metal oxide such as alumina, titania, silica, zirconia and hydroxyapatite, and a natural or synthetic mineral such as kaolin, montmorillonite, manganese oxide and mica.

The organic compound may be granulated as the material itself if possible, or may be bonded onto microparticles of an organic or inorganic binding carrier by electrostatic adsorption or chemical binding and made to particles. In other words, the microparticle in the present invention may be formed by binding an adsorption material selectively adsorbing the physiologically active substance onto the surface of the carrier microparticle.

The adsorption material in this case may be one or multiple materials selected from PyroSep, polymixin B, polylysine, polyornithine, polyarginine, polyhistidine, aminosilane, chitosan, an anti-endotoxin antibody, an anti-endotoxin aptamer and a material in a random peptide library.

In addition, examples of the organic carrier microparticle in this case include microparticles of polystyrene latex, polyethylene, nylon, cellulose, agarose, polyvinyl alcohol, an acrylic resin and the like. In addition, examples of the inorganic carrier microparticle include microparticles of simple substances such as alumina, titania, silica, zirconia and hydroxyapatite, or composite-material microparticles thereof (for example, silica alumina, alumina titania and the like), and microparticles of a natural or synthetic mineral such as kaolin, montmorillonite, manganese oxide and mica, and the like.

These microparticles can be desirably isolated and collected with spontaneous sedimentation or a simple operation. It is difficult to collect microparticles having a very small particle diameter. Conversely, if the microparticles have a large particle diameter, (1) a dispersion property of the microparticles in water or an extraction liquid is low. (2) Effective surface area for adsorbing the microparticles is low. (3) It is difficult for the predetermined physiologically active substance to invade into the inside of the particle. For such reason, the adsorption ability for the predetermined physiologically active substance decreases. (4) In the case where the microparticle after having adsorbed the predetermined physiologically active substance should be washed, water in the inside of the microparticle is hardly discharged, and thus the washing effect decreases. Furthermore, in reacting the microparticle having adsorbed the predetermined physiologically active substance with AL to induce the co-aggregation, smaller particle diameter as possible leads to shortening of the measurement time. From these, the particle diameter of the microparticles is desirably 5 nm to 50 µm, and further desirably 10 nm to 20 µm.

In addition, the predetermined fluorescent dye in this case is not particularly limited, but examples thereof include fluoresceins, rhodamines, coumarins, pyrenes and cyanines similarly to the case where the AL-bound bead reagent is used. These fluorescent dyes may be suitably used.

In addition, as the predetermined fluorescent dye, 4',6-diamidino-2-phenylindole (DAPI), umbelliferone, 9,10-bis(phenylethynyl)anthracene, 2-chloro-9,10-bis(phenylethynyl)anthracene, SYBR Green (SG), ethidium bromide, stilbene, Nile blue (Nile blue A), 5,12-bis(phenylethynyl)naphthacene, pyranine, perylene, boron-dipyrromethene, merocyanine, green fluorescent protein (GFP), luciferin, rubrene, Lumazine protein (LumP) and the like may be used. In addition, fluorescein isothiocyanate (FITC), rhodamine isothiocyanate (RITC), QUARTZ DOT (trademark) that is a fluorescent dye in which an organic dye molecule is immobilized in silica (silicon dioxide) particles, or the like may be used.

In addition, also in this case, the inorganic fluorescent dye group described above may be used to be bonded to the substance involved in the aggregation or gelation, and in addition, may be also used as the microparticle itself emitting fluorescence. Accordingly, by forming the microparticle with the inorganic fluorescent dye group, it is possible to impart a function of generating a predetermined fluorescence to the microparticle.

In addition, the physiologically active substance of biological origin in the present invention may be endotoxin or β glucan. Then, it is possible to perform more accurate detection or concentration-measurement of endotoxin that is the most representative pyrogenic substance, and suppress entry of a transfusion, a medicine for injection, the blood and the like contaminated with endotoxin into the human body to induce side effects. Similarly, it is possible to perform more accurate detection or concentration-measurement of β glucan, and perform more accurate screening of infectious diseases due to a variety of fungi including not only fungi which are frequently observed in general clinical practices such as Candida, Aspergillus, and Cryptococcus, but also rare fungi.

Furthermore, the means for resolving the problems of the present invention described above can be used in combination as possible.

### Advantageous Effects of Invention

According to the present invention, provided is a method for measuring a physiologically active substance of biological origin by producing a mixed solution of an AL reagent containing AL which is a hemocyte extract of limulus and a sample containing a predetermined physiologically active substance of biological origin, and detecting protein aggregation or gelation induced by the reaction between AL and the physiologically active substance in the mixed solution while stirring the mixed solution, whereby to detect the physiologically active substance in the sample or measure the concentration of the physiologically active substance, which makes it possible to perform more accurate detection or concentration-measurement of the physiologically active substance of biological origin, and to visually observe the process of the protein aggregation or gelation induced by the reaction between AL and the physiologically active substance.

### Brief Description of Drawings

Fig. 1 is a figure illustrating the schematic constitution of the fluorescence measurement apparatus in Examples of the present invention.
Fig. 2 is a graph illustrating the temporal change of the light transmittance in the case where endotoxin was measured with the fluorescent AL-bound bead method in Examples of the present invention.
Fig. 3 is a figure illustrating the temporal change of the fluorescence distribution in the case where endotoxin was measured with the fluorescent AL-bound bead method in Examples of the present invention.
Fig. 4 is an example of an image of the fluorescence distribution at a predetermined time in the case where endotoxin was measured with the fluorescent AL-bound bead method in Examples of the present invention.
Fig. 5 is a figure illustrating the temporal change of the fluorescence intensity in the case where endotoxin was measured with the fluorescent AL-bound bead method in Examples of the present invention.
Fig. 6 is a graph illustrating the temporal change of the light transmittance in the case where endotoxin-adsorptive microparticles bound with endotoxin, and further bound with a fluorescent dye, and mixed with the AL reagent in Examples of the present invention.
Fig. 7 is a figure illustrating the temporal change of the fluorescence distribution in the case where endotoxin-adsorptive microparticles bound with endotoxin, and further bound with a fluorescent dye, and mixed with the AL reagent in Examples of the present invention.

### Description of Embodiments

Hereinafter, embodiments of the present invention are specifically illustrated using the figures. Furthermore, in the description below, endotoxin is taken as examples and explained as the predetermined physiologically active substance, but the description below can be also applied to other physiologically active substances of biological origin including β glucan.

In the present embodiment, the substance involved in the aggregation or gelation of a coagulin (for example, proteins such as a coagulogen in AL, the AL-bound beads, the microparticles bound with endotoxin, and the like) is imparted in advance with a function of generating a predetermined fluorescence when the measurement of endotoxin is performed by detecting aggregation or gelation of a coagulin produced by the reaction between endotoxin and AL. More specifically, the predetermined fluorescent dye is bonded to the substance involved in the aggregation or gelation such as a coagulin, or the AL-bound bead or the microparticle bound with endotoxin is formed with the inorganic fluorescent dye. Then, when the protein aggregation or gelation is generated, the intensity of the fluorescence from the fluorescent dye or the bead or the microparticle emitting fluorescence itself is measured, or the fluorescence distribution is observed. This makes it possible to visually observe the mechanism of the aggregation or gelation of a coagulin. In addition, since the fluorescence intensity increases at a site where the aggregation or gelation is generated, measurement of the fluorescence intensity enables detection the protein aggregation or gelation.

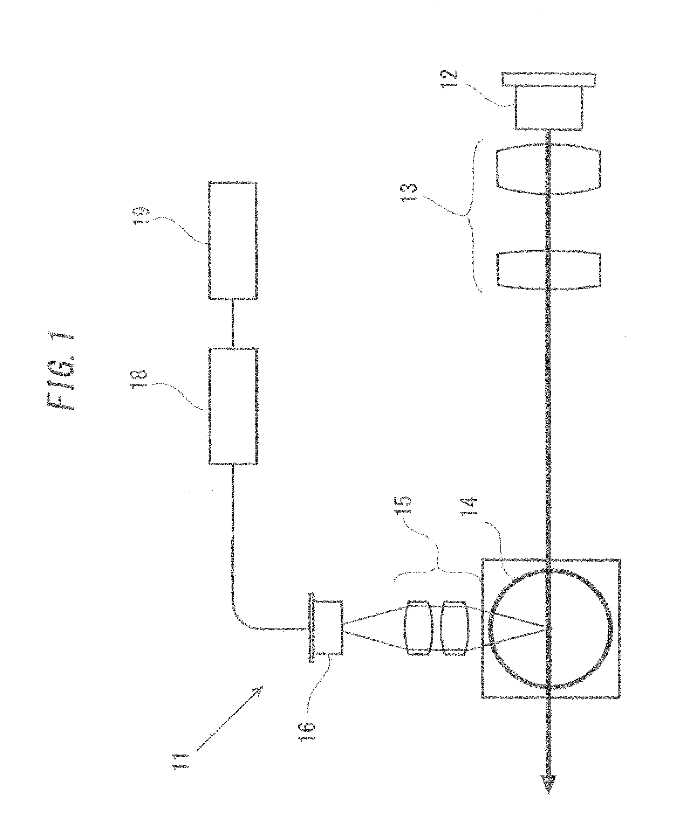

Fig. 1 illustrates one example of the schematic constitution of a fluorescence measurement apparatus 11 in the present embodiment. This fluorescence measurement apparatus 11 is an apparatus for sampling a sample under measurement for endotoxin at a predetermined time with the fluorescent AL-bound bead method, for example, using a device for turbidimetric measurement, and measuring the fluorescence distribution in the mixed solution at the time. As an exciting light source 12 used in the fluorescence measurement apparatus 11, an argon laser light source having 488 nm wavelength is used. However, an exciting light source constructed by combining an argon laser light source, and in addition, a Xenon lamp, a filter and a dichroic mirror may be also used. The exciting light emitted from the light source 12 is narrowed with an incidence optical system 13, and enters a sample cell 14. In this sample cell 14, a mixed solution of the sample and the AL reagent in the fluorescent AL-bound bead method is kept. The light having entered the sample cell 14 excites fluorescence in the particle adsorbed with the fluorescent dye in the mixed solution (alumina bead and coagulogen or coagulin changed from coagulogen).

An output optical system 15 is positioned on the lateral side of the incident optical axis of the sample cell 14. In this output optical system 15, fluorescence having 535 nm of the wavelength in the case where the fluorescent dye is fluorescein isothiocyanate (hereinafter, also referred to as FITC.) or fluorescence having 580 to 600 nm of the wavelength in the case where the fluorescent dye is rhodamine isothiocyanate (hereinafter, also referred to as RITC.) is emitted. In addition, on the extension of the optical axis of the output optical system 15, an imaging element 16 is positioned which is generated from the particle in the mixed solution in the sample cell 14, and in which the fluorescence distribution image in the sample cell 14 is imaged with the output optical system 15. The imaging element 16 is electrically connected with an arithmetic unit 18 for calculating a total value of the fluorescence intensity in the image from the image imaged on the imaging element 16, and a display unit 19 for displaying the image imaged on the imaging element 16.

### (Preparation Example 1)

Next, Preparation Example of the fluorescent AL-bound bead in which a red fluorescent dye is bonded to the bead will be explained. As the bead, an alumina bead ASFP-20 (20 nm bead diameter) manufactured by DENKI KAGAKU KOGYO KABUSHIKI KAISHA was used. In addition, as the red fluorescent dye, RITC was used. When binding the red fluorescent dye to the bead, a RITC solution was mixed with a suspension liquid in which alumina beads were dispersed, and the mixture was stirred whereby to adsorb RITC to the bead.

In addition, in this Preparation Example, a green fluorescent dye was bonded to a coagulogen that is a protein contained in AL, and the coagulogen tagged with the green fluorescent dye was adsorbed onto the bead tagged with the red fluorescent dye. At the time, a solution in which FITC was dissolved in dimethyl sulfoxide (hereinafter, also referred to as DMSO.), which is an amphiphilic solvent, was mixed with an AL reagent (ES-II Multi reagent manufactured by Wako Pure Chemical Industries, Ltd.) and stirred, and extra DMSO and FITC were removed by cold ethanol method, whereby to bind the amino group of the coagulogen in the AL reagent to the isothiocyanate in FITC, and thus prepare an AL reagent bound with the green fluorescent dye.

Then, the alumina beads ASFP-20 adsorbed with RITC were put into a glass tube in subdivision and the openings of the glass tubes were covered with an aluminum foil. Further, several pieces were collected and put into a glass beaker, and the openings were further covered with an aluminum foil, hot-air treated for 30 minutes at 250°C whereby to completely inactivate endotoxin which may contaminate the beaker. This was dispersed in a dispersion liquid to the predetermined concentration whereby to prepare beads having no endotoxin selectivity but having adsorption ability.

Further, the alumina beads ASFP-20 bound with RITC were dispersed in distilled water for injection in 30 mg/mL of the concentration. Then, 500 µL of the dispersion liquid of ASFP-20 adsorbed with RITC was added to 500 µL of the AL reagent (ES-II Multi reagent manufactured by Wako Pure Chemical Industries, Ltd.) adsorbed with FITC, and well mixed. Then, the mixture was washed once with water for injection, and dispersed in 1 mL of water for injection, to give alumina beads adsorbed with RITC, and bound with coagulogen adsorbed with FITC (the fluorescent AL-bound bead).

Furthermore, in Example, RITC was adsorbed to the alumina beads. However, a method may be also used in which a fluorescent dye (RITC or FITC) is previously bonded to aminosilane (binding the isothiocyanate group to the amino group of aminosilane), and then the fluorescent dye-bonded silane is immobilized onto the bead using the coupling action of silane.

### (Preparation Example 2)

Next, a preparation example of microparticles bound with endotoxin and a fluorescent dye (hereinafter, also referred to as the fluorescent endotoxin-bound microparticle.) will be explained. In this Preparation Example, 100 µL of the titania nanoparticles (P25: 100 mg/mL) as the microparticle, was mixed with 100 µL of FITC dispersed in DMSO (1 mg/mL). Then, the mixture was stirred for 30 minutes whereby to adsorb FITC onto the titania nanoparticle. Next, the titania nanoparticles adsorbed with FITC was washed three times with distilled water for injection and isolated, and then suspended in 1 mL of distilled water for injection.

Next, 100 µL of the suspension liquid containing the microparticles was mixed with 900 µL of a poly-L-lysine (0.1%) solution, and the mixture was stirred for 1 hour at room temperature whereby to adsorb poly-L-lysine onto the microparticle. Then, the microparticles were washed three times with distilled water for injection and isolated. Further, the microparticles were suspended in a 0.2% Triton X-100 solution in 0.2 mg/mL of the microparticle concentration. Then, 100 µL of the suspension liquid was mixed with 1 mL of endotoxin solution (0.001 EU/mL), and the mixture was stirred and reacted for 20 minutes. Then, the resultant was centrifuged to collect the microparticles, and the obtained microparticles were dispersed in 50 µL of a 0.02% Triton X-100 solution.

### (Example 1)

First of all, the change of the light transmittance when the reagent and the sample containing endotoxin were mixed, was measured with the stirring turbidimetric method using the fluorescent AL-bound bead obtained in Preparation Example 1 (using EX-100 manufactured by Kowa Company Ltd.). In this measurement, the fluorescent AL-bound beads obtained in Preparation Example 1 were suspended in physiological saline for injection in 20 mL. 100 µL was taken from this, and was mixed with the AL reagent. Further, the mixture was mixed with 100 µL of an aqueous solution containing endotoxin in 0.01 EU/mL of the concentration to give 200 µL of the total volume. Then, the mixed solution was set in EX-100, and the change of the light transmittance after initiation of the stirring was measured.

In addition, for control, the measurement was also performed in the case where only a fluorescent AL reagent containing a coagulogen bound to the green fluorescence dye FITC prepared in Preparation Example 1 was used instead of the fluorescent AL-bound bead. Specifically, the fluorescent AL reagent was suspended in physiological saline for injection in 20 mL. 100 µL was taken from this suspension liquid, and was mixed with the AL reagent. Further, the mixture was mixed with 100 µL of an aqueous solution containing endotoxin in 0.01 EU/mL of the concentration to give 200 µL of the total volume. Then, the mixed solution was set in EX-100, and the change of the light transmittance after initiation of the stirring was measured.

The mixed solution was sampled at an appropriate time interval, and the fluorescence distribution was observed with the fluorescence measurement apparatus 11 in the case where the fluorescent AL-bound bead were used and in the case where the fluorescent AL reagent were used in the measurement of the light transmittance. In addition, the total fluorescence intensity in the picture observed with the fluorescence measurement apparatus 11 was acquired by calculation from the image-processing.

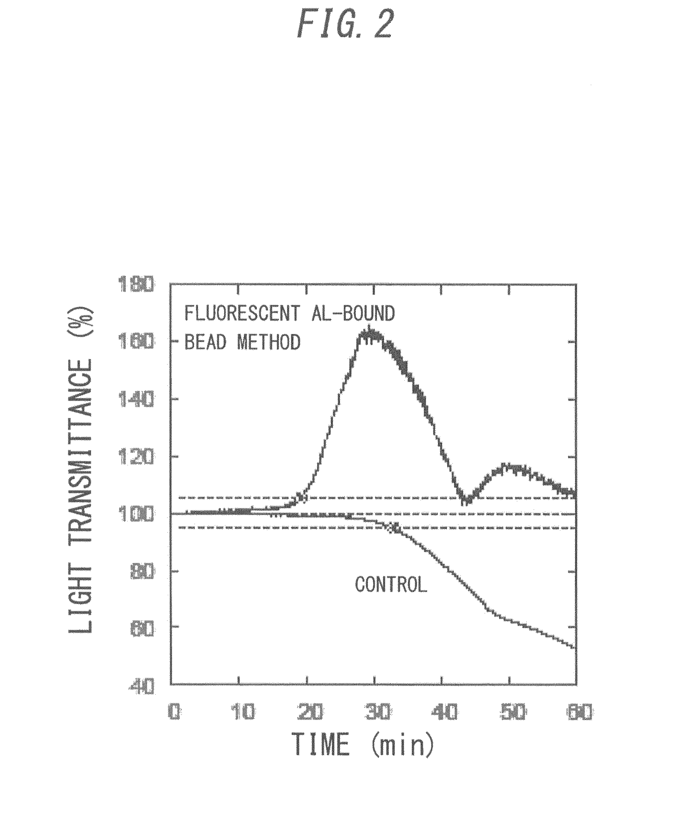

Fig. 2 illustrates the temporal change of the light transmittance of the mixed solution in the case where the fluorescent AL-bound beads were used, and in the case of Comparative Example (control) in which the fluorescent AL reagent was used. The horizontal axis represents the elapsed time after initiation of the stirring, and the vertical axis represents the light transmittance after initiation of the stirring when the light transmittance at the time of initiation of the stirring was taken as 100 (%). In addition, in this Example, the time point where the light transmittance changed by ±5% with respect to the initial value was defined as the aggregation initiation time.

From Fig. 2, it is understood that in the case of the control, the aggregation initiated and decrease of the light transmittance started from about 25 minutes elapsed after initiation of the stirring. On the other hand, it is understood that in the fluorescent AL bead method, conversely, the light transmittance increased rapidly from before 20 minutes elapsed, and rapidly decreased again in about 30 minutes elapsed. As understood from Fig. 2, in the fluorescent AL bead method, the aggregation initiation time is 17 to 18 minutes whereas the aggregation initiation time is about 32 to 33 minutes in the control.

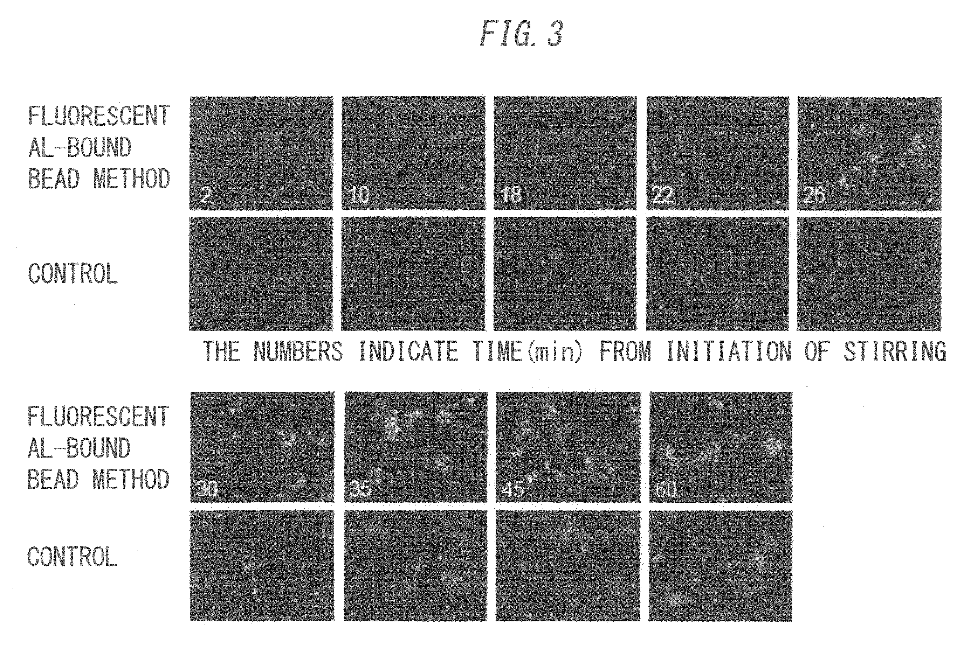

Next, Fig. 3 illustrates the temporal change of the image of the fluorescence distribution obtained in this measurement example. Each image illustrates the distribution of the fluorescence generated induced by aggregation of the beads and a coagulin changed from a coagulogen in the fluorescent AL-bound bead method, and the distribution of the fluorescence generated by aggregation of coagulin in Comparative Example. The numbers described in the image according to the fluorescent AL-bound bead method is the elapsed time from the initiation of the stirring. The image according to the control corresponding to the image according to the fluorescent AL-bound bead method is obtained at the same time as the corresponding image according to the fluorescent AL-bound bead method. In the fluorescent AL-bound bead method, the bright spot by fluorescence from the aggregated bead starts to be clear from the time position of 18 minutes elapsed corresponding to the time of rapid increase of the light transmittance in Fig. 2, and the fluorescence distribution by aggregation of coagulin around the core of the aggregated beads spreads after 30 minutes elapsed where the light transmittance reached the peak. Then, the fluorescence distribution further increases with elapse of the time.

On the other hand, it is understood that in the control, the time when the fluorescent bright spot appears is later in comparison to the fluorescence distribution according to the fluorescent AL-bound bead method, and the process of the spreading of the fluorescence distribution is also later in comparison to the fluorescent AL-bound bead method.

Furthermore, as illustrated in Fig. 3, in the fluorescent AL-bound bead method, red bright spots already appear at a stage of 2 minutes after initiation of the stirring, and the number of the bright spots increase with elapse of the time although it is difficult to distinguish them since Fig. 3 is a black-and-white photograph. Herein, it is understood that the bright spot appearing until 20 minutes is mainly based on the red fluorescent dye, and the aggregation mass of the fluorescent AL-bound beads is first generated and the fluorescence is emitted. Then, it was possible to observe the mechanism in which the process of the spreading of the fluorescence distribution is mainly based on the green fluorescence dye, and aggregation by coagulin is generated around the aggregation mass of the beads, which cross-links and wraps up the aggregation mass of each bead.

In addition, from the change of the light transmittance of Fig. 2 and the fluorescence distribution of Fig. 3 relating to the control, it is understood that the light transmittance does not change until 20 minutes or so elapsed after the stirring, but the bright spot appears from 2 minutes elapsed after the stirring in the fluorescence distribution, and the number of the bright spots increases before the elapse of 20 minutes. As described above, by visually observing the fluorescence distribution, it is possible to obtain information not obtained with the measurement of the light transmittance.

Next, from the fluorescence distribution obtained in the method, a method for quantifying the aggregation of the beads in the fluorescent AL-bound bead method will be explained.

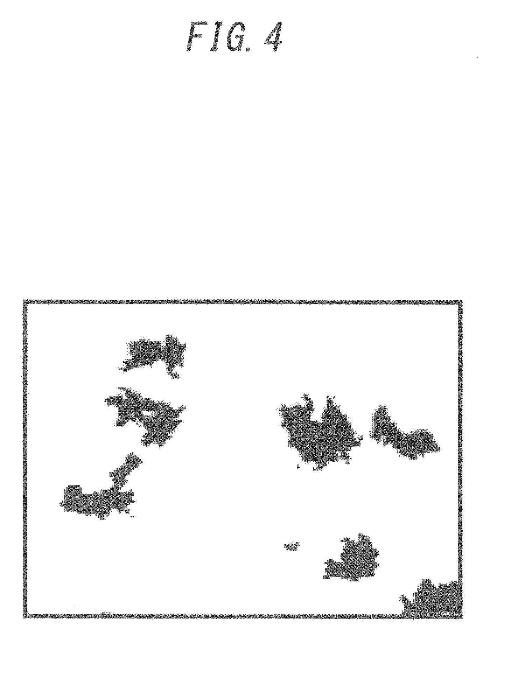

Fig. 4 illustrates the image of the fluorescence distribution clarified by image-processing the fluorescent image obtained in Fig. 3, and displaying the site having received the fluorescence in black color. This image is obtained from the fluorescent image at 30 minutes after initiation of the reaction in Fig. 3. Then, for example, by integrating the fluorescence intensity in the image of Fig. 4 and taking the value as the fluorescence intensity at this time, it is possible to quantify the aggregation of the beads in the fluorescent AL-bound bead method.

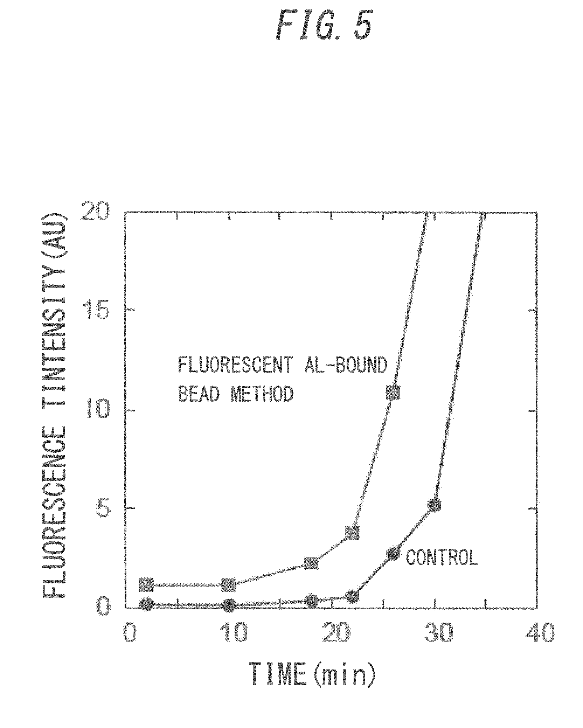

Fig. 5 illustrates the changes of the fluorescence intensity in the fluorescent AL-bound bead method and in the control when the time from initiation of the stirring was taken on the horizontal axis, and the fluorescence intensity (integration value thereof) is taken on the vertical axis. According to this, it is understood that in the fluorescent AL-bound bead method, the fluorescence intensity rapidly increases after 17 to 18 minutes elapsed similarly to the measurement by the light transmittance. In addition, it is understood that higher fluorescence intensity is obtained according to the fluorescent AL-bound bead method in comparison to the control. As described above, it is understood that according to the fluorescent AL-bound bead method, it is possible to measure endotoxin in a shorter time with high sensitivity.

Furthermore, in Preparation Example 1 and Example 1, a fluorescent dye was bonded to both of the bead and the protein adsorbed onto the surface of the bead, and aggregation or gelation induced by the reaction between endotoxin and AL of the fluorescent AL-bound bead was measured. However, in the present invention, the fluorescent dye may be bonded only to the bead, or the bead itself may be formed with a material emitting fluorescence by itself, and the fluorescent AL-bound bead method may be implemented. This is because the mechanism of the aggregation or gelation can be sufficiently observed with the fluorescence emitted from the beads only, and also the fluorescence intensity sufficient for performing the measurement of endotoxin can be obtained. According to this, it is possible to omit an operation for binding the fluorescent dye to the protein, which is the most complicated process, and more easily perform the measurement of endotoxin by fluorescence.

### (Example 2)

Next, using the fluorescent endotoxin-bound microparticles obtained in Preparation Example 2, first of all, the change of the light transmittance when a sample containing the fluorescent endotoxin-bound microparticle was mixed with the AL reagent, was measured by the stirring turbidimetric method (using EX-100, manufactured by Kowa Company Ltd.). In this measurement, 50 µL of the dispersion liquid in which the fluorescent endotoxin-bound microparticles obtained in Preparation Example 2 were dispersed, was mixed with 50 µL of the AL reagent to give 100 µL of the mixture. Then, the change of the light transmittance after initiation of the stirring was measured with EX-100. In addition, the mixed solution under the measurement of the light transmittance was sampled at an appropriate time interval and the fluorescence distribution was observed with the fluorescence measurement apparatus 11.

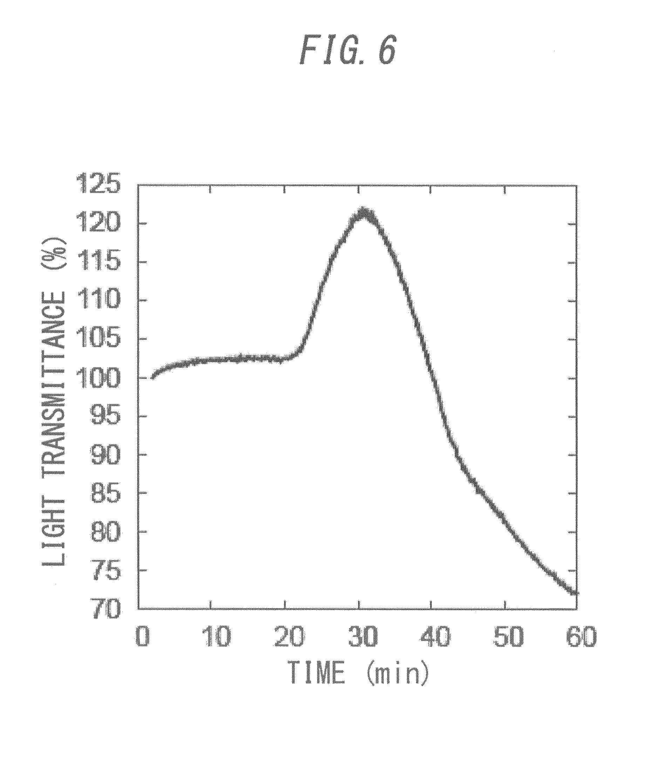

Fig. 6 illustrates the temporal change of the light transmittance in the case where the fluorescent endotoxin-bound microparticles were used (hereinafter, also referred to as the "fluorescent endotoxin-bound microparticle method".). The horizontal axis represents the elapsed time after initiation of the stirring, and the vertical axis represents the light transmittance after initiation of the stirring when the light transmittance at the time of initiation of the stirring was taken as 100 (%). From Fig. 6, it is understood that also in the fluorescent endotoxin-bound microparticle method, the light transmittance increased rapidly from after 20 minutes elapsed from initiation of the stirring, and rapidly decreased again in about 30 minutes elapsed.

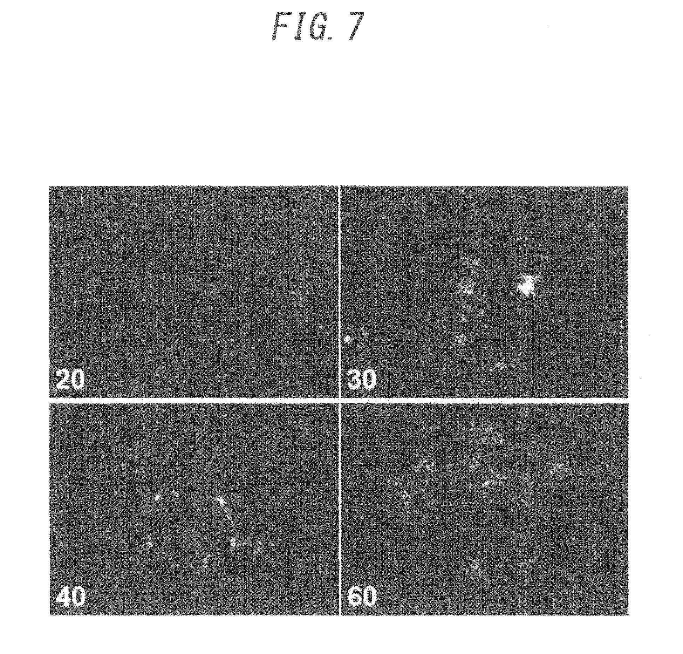

Next, Fig. 7 illustrates the image of the fluorescence distribution obtained by the present measurement examples. Each image illustrates the fluorescence distribution induced by aggregation of the microparticles in the fluorescent endotoxin-bound microparticle method. It is understood that also in the fluorescent endotoxin-bound microparticle method, the bright spot by fluorescence starts to be clear from the time position of 20 minutes elapsed corresponding to the time of rapid increase of the light transmittance in Fig. 6, and the fluorescence distribution spreads after 30 minutes elapsed where the light transmittance reaches the peak. Then, the fluorescence distribution further spreads with elapse of the time.

### Reference Signs List

- 11: Fluorescence measurement apparatus
- 12: Exciting light source
- 13: Incident optical system
- 14: Sample cell
- 15: Output optical system
- 16: Imaging element
- 18: Arithmetic unit
- 19: Display unit

## Claims

1. A method for measuring a physiologically active substance of biological origin by producing a mixed solution of an AL reagent containing AL which is a hemocyte extract of limulus and a sample containing a predetermined physiologically active substance of biological origin, and detecting protein aggregation or gelation induced by the reaction between AL and the physiologically active substance in the mixed solution while stirring the mixed solution, whereby to detect the physiologically active substance in the sample or measure the concentration of the physiologically active substance, which is characterized that
imparting a function of generating a predetermined fluorescence to a substance involved in the aggregation or gelation in the sample and/or the AL reagent, and
measuring or observing the fluorescence from the substance involved in the aggregation or gelation in the mixed solution whereby to detect the aggregation or gelation in the mixed solution and thus detect the physiologically active substance in the sample or measure the concentration of the physiologically active substance.

2. The method for measuring a physiologically active substance of biological origin according to claim 1 by
binding the predetermined protein contained in the AL to a bead dispersible in the AL reagent,
mixing AL-bound bead reagent which is an AL reagent in which the beads bound with the protein are dispersed together with the sample, and
detecting the protein aggregation or gelation in the mixed solution of the AL-bound bead reagent and the sample whereby to perform detection or concentration measurement of the physiologically active substance, which is characterized that
a predetermined fluorescent dye is previously bonded to the bead, or the bead is formed with the predetermined inorganic fluorescent dye, whereby to impart a function of generating a predetermined fluorescence to the bead.

3. The method for measuring a physiologically active substance of biological origin according to claim 1 or 2, which is characterized that a predetermined fluorescent dye is bonded to a protein that is aggregated or gelated due to the reaction between AL and the physiologically active substance in the mixed solution, whereby to impart a function of generating a predetermined fluorescence to the protein.

4. The method for measuring a physiologically active substance of biological origin according to any one of claims 1 to 3, which is characterized that the predetermined protein is coagulogen purified from a hemocyte extract of limulus.

5. The method for measuring a physiologically active substance of biological origin according to any one of claims 1 to 4
dispersing microparticles capable of adsorbing the physiologically active substance in the sample whereby to adsorb the physiologically active substance onto the microparticles and then isolating the microparticles from the sample,
producing a mixed solution of the AL reagent and the microparticle adsorbed with the physiologically active substance, and
detecting aggregation or gelation of the microparticles in the mixed solution of the AL reagent and the microparticles, which is characterized that
a predetermined fluorescent dye is bonded to the microparticle in advance, or the microparticle is formed with a predetermined inorganic fluorescent dye, whereby to impart a function of generating a predetermined fluorescence to the microparticle.

6. The method for measuring a physiologically active substance of biological origin according to any one of claims 1 to 5, which is characterized that the physiologically active substance of biological origin is endotoxin or β glucan.
